# EUROPÄISCHE PATENTSCHRIFT

(11) **EP 1 749 504 B1**
(45) Veröffentlichungstag und Bekanntmachung des Hinweises auf die Patenterteilung: **15.02.2012**
(21) Anmeldenummer: 06015734.4
(22) Anmeldetag: 28.07.2006
(51) Int. Cl.: A61F 2/08, A61B 17/04

(54) **Ankerelement zum Fixieren einer Sehne**
Anchor element for the fixation of a ligament
Élément d'ancrage pour la fixation de ligament

(30) Priorität: 05.08.2005 DE 102005039080
(43) Veröffentlichungstag der Anmeldung: 07.02.2007
(73) Patentinhaber: Karl Storz GmbH & Co. KG, 78532 Tuttlingen (DE)
(72) Erfinder: Collin, Philippe, Dr., 35590 Clayes (FR); Berberich, Sascha, 78532 Tuttlingen (DE)
(74) Vertreter: Weller, Wolfgang

(56) Entgegenhaltungen:
- WO-A-98/26717
- DE-U1- 20 007 777
- DE-U1- 29 717 413
- US-A- 5 782 866
- US-A- 6 139 565
- US-A- 6 149 653

## Beschreibung

Die Erfindung betrifft ein Ankerelement zum Einbringen in einen Knochen mit zumindest einem Faden zum Fixieren einer Sehne, eines Bandes oder von Weichteilen, mit zumindest einer Öffnung zum Aufnehmen des zumindest einen Fadens, und einer zentralen Bohrung zum Aufnehmen eines Werkzeuges zum Einbringen des Ankerelementes, wobei die zentrale Bohrung in einem proximalen Abschnitt des Ankerelementes von einem materialstarken Mantel umgeben ist, wobei das Ankerelement als langgestreckter zylindrischer Körper, der keinen erweiterten Kopf aufweist, ausgebildet ist und der in einem distalen Abschnitt in eine Verjüngung übergeht.

Ein derartiges Ankerelement ist aus der US3,782,866 A2 bekannt.

Unterhalb des Abschnittes des Ankerelements, in dem die zentrale Bohrung zum Aufnehmen eines Werkzeuges zum Einbringen des Ankerelementes vorhanden ist, ist eine Öffnung in Form eines seitlich sich nach oben krümmenden Schlitzes eingeschnitten. Dabei erstreckt sich der Schlitz so weit in den massiven Körper hinein, dass ein in den Schlitz eingelegter Faden auf der Mittellängsachse des Ankerelements zum Liegen kommt.

Aus der DE 200 07 777 U1 ist ein Faden-Anker-System zum Verbinden von Gewebeteilen bekannt, bei dem ein Ankerelement eine zentrale, durch das Ankerelement vollständig hindurchgehende Öffnung aufweist, durch die ein Zieldraht zum Einbringen hindurchgeschoben werden kann. Im Bereich der distalen Spitze ist seitlich ein Schlitz eingeschnitten, in den während der Montage ein Faden so weit eingelegt werden kann, bis er auf den mittigen Zieldraht trifft. Nach dem Setzen des Ankerelements im Knochen kann der Zieldraht abgezogen und der Faden kann dann in den dadurch frei werdenden Raum weiter innen in das Ankerelement eingezogen und dort fixiert werden.

Diese Ankerelemente, auch als Fadenanker bezeichnet, dienen in der Chirurgie dazu eine abgerissene Sehne, ein Band oder ein anderes Gewebe mit Hilfe eines Fadens wieder am Knochen zu fixieren. Diese können auch zur Fixierung von Weichteilgewebe, eine sogenannte Labrumrefixation, eingesetzt werden.

Für die Ausgestaltung der Ankerelemente steht es mit an vorderster Stelle, dass ein Faden zur Fixierung der abgerissenen Sehne vom eingebrachten Ankerelement sicher gehalten wird. Dies setzt entsprechende Überlegungen für das Gestalten einer Fadenaufnahme im Ankerelement voraus.

Eines der Haupteinsatzgebiete für solche Ankerelemente ist das Fixieren abgerissener Sehnen im Schulterbereich.

Ein Beispiel für ein derartiges Ankerelement ist in den Abbildungen 8A bis 8F der oben genannten US 6,139,565 dargestellt.

Das dort gezeigte Ankerelement weist einen langgezogenen zylindrischen Körper mit einer zentralen Bohrung auf. Diese zentrale Bohrung ist als Kanal mit einem quadratischen Querschnitt axial durch das Ankerelement hindurchgeführt und dient zur Aufnahme des Drehwerkzeuges zum Eindrehen des Ankerelementes in den Knochen.

An einem proximalen Abschnitt des Ankerelementes stehen kreuzförmig um die Öffnung des Kanals vier Stege vor, in denen jeweils eine Fadenöse ausgenommen ist.

Da die Fadenösen der Aufnahme und dem Halt des Fadens dienen sollen, ist es als nachteilig anzusehen, dass die Fadenösen in proximal vorstehenden Stegen ausgespart sind. Die Stege als solche stellen schon materialschwache Abschnitte des Körpers des Ankerelementes dar, die durch die Fadenösen weiter geschächt sind.

Der Faden wird in den schmalen Fadenösen des Ankerelementes eingefädelt.

Nachdem das Ankerelement gesetzt ist, wird das Drehwerkzeug abgenommen. Sodann wird die Sehne mit den aus der Bohrung vorstehenden Enden des Fadens fixiert. Sowohl beim Fixieren als auch postoperativ wirken starke Kräfte auf die Fadenöse ein. Bei Fadenösen in schmalen materialschwachen Stegen besteht die Gefahr, dass durch Materialausbrüche der Faden sich vom Ankerelement löst. Schmale Stege stellen geringflächige Berührungsstellen mit dem Faden dar, die durch Bewegungen ein Durchscheuern des Fadens fördern.

Da in der modernen Medizin auch Ankerelemente aus resorbierbaren Materialien eingesetzt werden, die nach und nach durch körpereigenes Knochenmaterial ersetzt werden können, ergibt sich durch die materialschwache Ausführung der Fadenöse ein Problem.

Dies besteht in der Gefahr von Ausreißen oder Ausbrechen der Fadenöse, was besonders im späteren Verlauf während des Heilungsprozesses zu schweren Komplikationen führen kann.

Es ist daher Aufgabe der vorliegenden Erfindung, ein Ankerelement der eingangs genannten Art zu schaffen, das eine verbesserte Stabilität für eine Fadenaufnahme aufweist.

Erfindungsgemäß wird die Aufgabe dadurch gelöst, dass im proximalen Abschnitt des zylindrischen Körpers, in dem der Mantel die zentrale Bohrung umgibt, im Mantel zwei diametral angeordnete Schlitze ausgebildet sind, die sich zur radialen Außenseite öffnen und sich in umfängliche Richtung im Mantel erstrecken, und dass deren Flanken radial nach außen gesehen, nach distal geneigt sind, wobei die zentrale Bohrung und die Schlitze räumlich getrennt sind.

Im Sinne der Erfindung ist unter einer "zentralen Bohrung" sowohl eine Sacklochbohrung als auch ein durchgehender Kanal zu verstehen.

Unter dem Begriff "materialstarker Mantel" ist ein Abschnitt des Ankerelementes zu verstehen, der proximal die zentrale Bohrung umgibt. Dieser Abschnitt ist ungeschwächt durch Materialreduktionen und ist dadurch besonders gut für eine Aufnahme von Fadenführungen geeignet.

Eine Fadenaufnahme in derartig ausgestalteten Schlitzen bietet dem Faden eine entsprechend große Anlagefläche. Diese Anlagefläche erstreckt sich in einem Teilumfang des materialstarken Mantels des Ankerelementes. Dadurch leitet der Faden die an ihm auftretenden Kräfte an einen massiven Abschnitt des Ankerelementes ab.

Je größer der Teilumfang ist, in dem der Faden im Ankerelement aufgenommen ist, desto größer ist folglich auch die Anlagefläche des Fadens. Dadurch werden die am Faden auftretenden Kräfte gleichmäßig über einen größeren Bereich an das Ankerelement abgeleitet und lokal konzentrierte Kräfte vermieden. Dies schützt die Fadenaufnahme vor einem Ausreißen oder Ausbrechen, und schont auch den Faden bei Relativbewegungen zwischen Faden und verankertem Ankerelement.

Weiterhin bietet diese Ausgestaltung den Vorteil, dass die Fadenaufnahme und der Verlauf einer zentralen Bohrung im Ankerelement voneinander räumlich getrennt gehalten werden.

Dies erlaubt es die zentrale Bohrung in ihrer Tiefe an verschieden ausgeführte Drehwerkzeuge anzupassen und zwar unabhängig von der Ausgestaltung der Fadenöse. Dadurch ist eine optimale Eindringtiefe eines Werkzeuges in das Ankerelement gegeben.

Darüberhinaus ist ein Einlegen des Fadens erleichtert, da dieser nur in den jeweiligen Schlitz eingeschoben zu werden braucht. Ein Verheddern von Faden und Drehwerkzeug ist ausgeschlossen.

Zwei Schlitze haben den Vorteil, dass im Ankerelement mehrere Fäden aufnehmbar sind. Hierdurch werden durch Zug entstehende Kräfte auf verschiedene Fäden und Stellen im Ankerelement verteilt und dadurch verringert. Daher werden diese Zugkräfte noch gleichmäßiger an das Ankerelement abgeleitet.

Zwei Schlitze im Ankerelement stellen einen sehr guten Kompromiss zwischen den bisher genannten Vorteilen und dem aus dem Ankerelement ausgenommenen Material dar.

Entsprechend der Erfindung sind die Schlitze diametral im Ankerelement angeordnet.

Die Maßnahme hat den Vorteil, dass ein Befestigen des Ankerelementes, mit Hilfe der in den Schlitzen eingehängten Fäden, am Werkzeug in einer gleichmäßigen Art erfolgt. Daher werden Kräfte, die während des Einbringens am Ankerelement auftreten gleichmäßig verteilt.

Außerdem sind sowohl das Ankerelement als auch die Fäden damit während des Einbringens verlustsicher am Werkzeug befestigt.

Diese Neigung hat den Vorteil, dass die Schlitze so im Ankerelement einbringbar sind, dass ein aufgenommener Faden, durch ein nach proximal gerichtetes Festziehen, einfach radial nach innen in den Schlitz hineingleitet und festsitzt. Ein einmal aufgenommener und gespannter Faden verbleibt im geneigten Schlitz des Ankerelementes. Solange der Faden gespannt im Ankerelement sitzt, ist daher eine sichere Aufnahme des Fadens gewährleistet.

Das Ankerelement, dass auf das Werkzeug aufgeschoben ist, lässt sich mit Hilfe des gespannten Fadens verlustsicher am Werkzeug befestigen. Dies gewährt das sichere Einbringen des Ankerelementes in den Knochen.

In einer Ausgestaltung der Erfindung weisen die Schlitze Flanken auf, die parallel verlaufen.

Diese Maßnahme hat den Vorteil, dass der Schlitz z.B. in einem aus Metall bestehenden Ankerelement durch ein Ausfräsen einfach herstellbar ist. Im Fall bioresorbierbarer Materialien ist ein solches Ankerelement einfach in einem Spritzgussverfahren herstellbar.

In einer weiteren Ausgestaltung der Erfindung weisen die Schlitze Flanken auf, die divergierend verlaufen.

Diese Maßnahme hat den Vorteil, dass sich der Schlitz durch die divergierenden Flanken zur radialen Außenseite hin verbreitert. Durch diesen verbreiterten Schlitz ist das Einbringen des Fadens erleichtert. Dabei sind die Flanken so im Ankerelement einbringbar, dass ein aufgenommener Faden, durch ein nach proximal gerichtetes Festziehen, einfach radial nach innen in den Schlitz hineingleitet und festsitzt, solange der Faden gespannt gehalten wird.

In einer weiteren Ausgestaltung der Erfindung sind die Flanken an ihren Kanten abgefast.

Diese Maßnahme hat den Vorteil, dass ein Abfasen der Kanten verhindert, dass der Faden durch Scheuern beschädigt oder aufgetrennt wird.

In einer weiteren Ausgestaltung der Erfindung weist die Neigung einen Winkel von etwa 10° bis etwa 80° auf.

Diese Maßnahme hat den Vorteil, dass der Schlitz in seiner Neigung je nach Einsatzzweck und Ausgestaltung des Ankerelementes unterschiedlich ausführbar ist.

Zum Einen erlaubt dies eine Neigung, die ausreichend steil ist, um einen aufgenommenen Faden selbst bei einer gelockerten Spannung noch sicher im Schlitz zu halten. Diese Variationen sind aufgrund des materialstarken Mantels möglich.

In einer weiteren Ausgestaltung der Erfindung weist der zumindest eine Schlitz einen abgerundeten Boden auf.

Diese Maßnahme hat den Vorteil, dass der Faden in einem gewissen Maß im Schlitz Querbewegungen durchführen kann, wobei ein Durchscheuern im abgerundeten Boden ausgeschlossen ist.

In einer weiteren Ausgestaltung der Erfindung weist der abgerundete Boden einen gekrümmten Verlauf auf.

Diese Maßnahme hat den Vorteil, dass der Faden in einem gekrümmt verlaufenden Boden über einen relativ langen Abschnitt des Schlitzes anliegt. Dies vermeidet lokale Anlagepunkte für den Faden. Damit werden auftretende Zugkräfte gleichmäßig an das Ankerelement abgeleitet.

In einer weiteren Ausgestaltung ist der gekrümmte Verlauf U-förmig.

Diese Maßnahme hat den Vorteil, dass ein U-förmiger Verlauf der Krümmung, eine optimale Kraftableitung für nach proximal gerichtete Zugkräfte am Faden an das Ankerelement erlaubt.

In der Ausgestaltung der Erfindung weist das Ankerelement zwei Schlitze auf.

Diese Maßnahme hat den Vorteil, dass zwei Schlitze im Ankerelement einen sehr guten Kompromiss zwischen den bisher genannten Vorteilen und dem aus dem Ankerelement ausgenommenen Material darstellen.

In der Ausgestaltung der Erfindung sind die Schlitze diametral im Ankerelement angeordnet.

Diese Maßnahme hat den Vorteil, dass ein Befestigen des Ankerelementes, mit Hilfe der in den Schlitzen eingehängten Fäden, am Werkzeug in einer gleichmäßigen Art erfolgt. Daher werden Kräfte, die während des Einbringens am Ankerelement auftreten gleichmäßig verteilt.

Außerdem sind sowohl das Ankerelement als auch die Fäden damit während des Einbringens verlustsicher am Werkzeug befestigt.

Weiterhin hat diese Maßnahme den Vorteil, dass auftretende Zugbelastungen an den eingelegten Fäden an gegenüberliegenden Stellen des eingebrachten Ankerelementes an den Knochen abgeleitet werden. Dadurch ist eine gleichmäßige Belastung des Ankerelementes in einer Belastungssituation im Knochen gewährleistet. Eine Beschädigung des Ankerelementes durch lokal begrenzt auftretende Spitzenkräfte wird dadurch ausgeschlossen.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: eine Seitenansicht eines erfindungsgemäßen Ankerelements,
- Fig. 2: einen Schnitt längs der Linie II-II in Fig. 1,
- Fig. 3: eine Seitenansicht des Ankerelementes von Fig. 1 sowie eines distalen Endes eines Werkzeuges, das von proximal nach distal in das Anker- element eingeschoben werden soll,
- Fig. 4: einen Zusammenbau des Ankerelementes und des Werkzeuges von Fig. 3 samt eingelegter Fäden, und
- Fig. 5: eine Situation beim Einbringen des Ankerelementes in einen Schulter- knochen mit Hilfe eines Zusammenbaus aus Ankerelement, Werkzeug und Fäden.

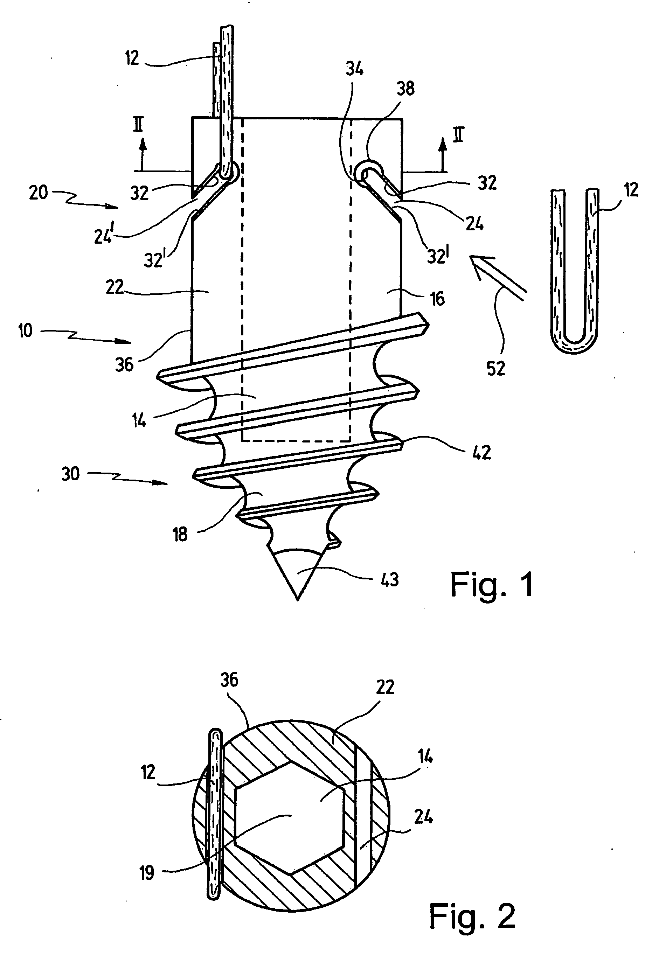

Das Ankerelement 10 der Fig. 1 und 2 weist in seinem proximalen Abschnitt 20 einen langgestreckten in etwa zylindrischen Körper 16 auf. Dieser zylindrische Körper geht in einem distalen Abschnitt 30 in eine Verjüngung 18 über.

Entlang der Verjüngung 18 verläuft nach distal ein selbstschneidendes Gewinde 42. Dieses selbstschneidende Gewinde 42 mündet in einer Spitze 43.

Das Ankerelement 10 weist in seinem proximalen Abschnitt 20 eine zentrale Bohrung 14 auf. Diese zentrale Bohrung 14 ist als Sacklochbohrung ausgeführt ist.

Diese zentrale Bohrung 14 erstreckt sich axial entlang der Mittellängsachse 19 des Ankerelementes 10. Sie reicht tief in das Ankerelement 10 hinein und weist, wie in Fig. 2 gezeigt, einen sechseckigen Querschnitt auf. Dieser dient einer drehschlüssigen Aufnahme eines Werkzeuges.

Dabei ist die zentrale Bohrung 14 von einem materialstarken Mantel 22 umfänglich umgeben.

In diesem materialstarken Mantel 22 sind zwei Schlitze 24 und 24' eingelassen. Sie verlaufen quer zur Mittellängsachse 19 des Ankerelementes 10. Dabei erstrecken sie sich gleichermaßen in umfänglicher Richtung des materialstarken Mantels 22.

Die Schlitze 24 und 24' weisen jeweils Flanken 32 und 32' auf. Diese Flanken 32, 32' verlaufen parallel nach Innen in das Ankerelement 10 und verbinden sich in einem abgerundeten Boden 34.

Nach Außen hin öffnen die parallelen Flanken 32, 32' jedes Schlitzes 24, 24' zu einer radialen Außenseite 36.

Sowohl die Flanken 32, 32' als auch der abgerundete Boden 34 sind an ihrem Übergang zur radialen Außenseite 36 abgefast.

Für eine sichere Fadenaufnahme sind die Flanken 32, 32' der Schlitze 24, 24', vom Boden 34 nach radial außen gesehen, nach distal geneigt.

Es ist dabei ersichtlich, dass ein in den Schlitz 24 bzw. 24' eingelegter Faden 12, der gespannt gehalten ist, nicht aus dem Schlitz 24 bzw. 24' nach distal hinausrutschen kann.

Fig. 2 veranschaulicht, dass die zentrale Bohrung 14 umfänglich vom materialstarken Mantel 22 umgeben ist. Eine Materialreduktion dieses materialstarken Mantels 20 ergibt sich nur durch die Schlitze 24 bzw. 24'.

Dabei ist auch ersichtlich, dass die zentrale Bohrung 14 und die Schlitze 24, 24' räumlich getrennt sind. Eine Aufnahme des Werkzeuges 44 findet somit ungestört von einer Fadenaufnahme im Ankerelement 10 statt.

Wie aus der Fig. 1 zu entnehmen ist, weist das Ankerelement 10, bestimmte Öffnungen in Form von Schlitzen, auf. In diesen sind Fäden 12 bzw. 12' aufnehmbar, so dass sich beide freie Fadenenden nach proximal vom Ankerelement 10, wegerstrecken. Die Fäden 12 bzw. 12' sind dabei jeweils diametral im Ankerelement 10, angeordnet.

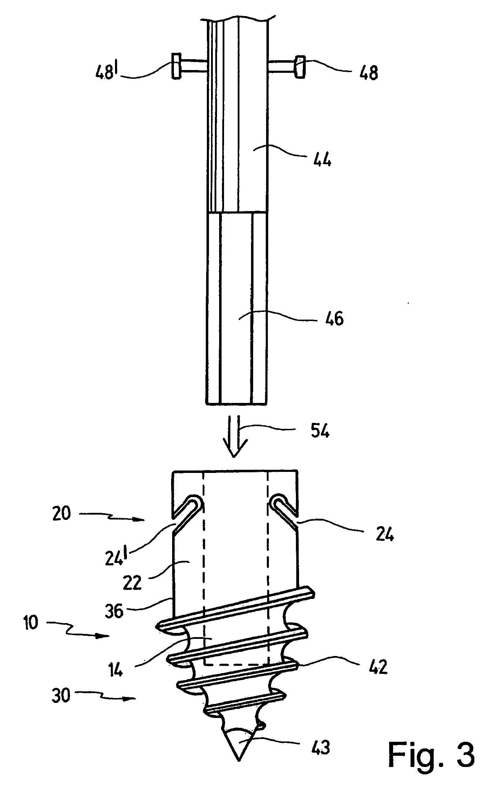

Aus Fig. 3 ist zu entnehmen, dass ein Werkzeug 44 in das Ankerelement 10 von proximal nach distal eingeschoben wird.

Das Werkzeug 44 weist ein sechseckiges distales Ende 46 auf. Der Querschnitt des distalen Endes 46 entspricht dem Querschnitt der zentralen Bohrung 14. Damit kann das distale Ende 46 in die zentrale Bohrung 14 eingeschoben werden wie in der Fig. 3 durch einen Pfeil 54 dargestellt.

Das distale Ende 46 ist damit im Ankerelement 10 dreh- und formschlüssig aufgenommen. Die Einschubtiefe des distalen Endes 46 ist der zentralen Bohrung 14 angepasst. Eine Behinderung durch etwaig querverlaufende Fäden 12 bzw. 12' ist in dieser Anordnung vermieden.

Wie aus der Fig. 3 weiterhin zu entnehmen ist, stehen radial vom Werkzeug 44 Zapfen 48 bzw. 48' vor. Diese sind diametral am Werkzeug 44 angeordnet und dienen dem Auffädeln bzw. Fixieren der Fäden 12, 12'.

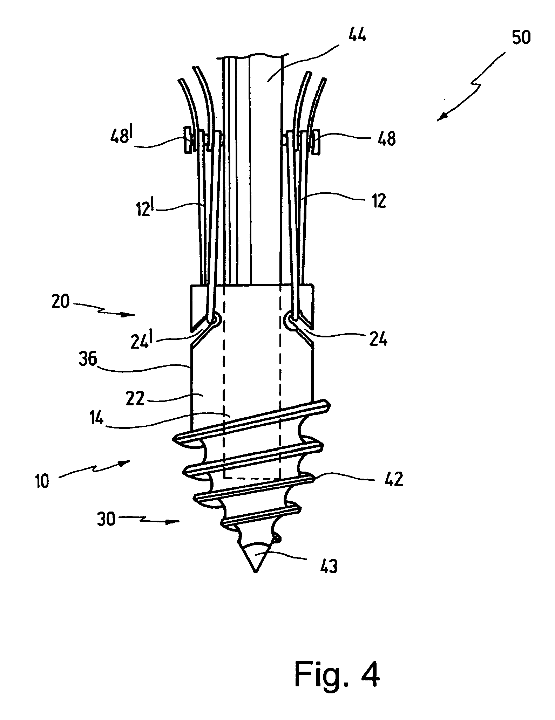

In der Fig. 4 ist ein vollständig in das Ankerelement 10 eingeschobenes Werkzeug 44 dargestellt.

Nun wird ein Faden 12 bzw. 12' in den jeweiligen Schlitz 24 bzw. 24' eingelegt, straff gespannt und über die Zapfen 48, 48' fixiert. Damit ist das Ankerelement 10 vor einem Verrutschen bzw. Verlust während des Einbringens gesichert.

Dies wird durch den sechseckigen Querschnitt der zentralen Bohrung 14 des jeweiligen Ankerelementes 10 unterstützt. Hierdurch ist eine form- und drehschlüssige Aufnahme des Werkzeuges 44 im Ankerelement 10 gegeben.

Ein aus Ankerelement 10, Fäden 12 bzw. 12' und Werkzeug 44 komplett zusammengesetztes Werkzeug 50 zum Einbringen eines Ankerelementes ist in der Fig. 4 gezeigt.

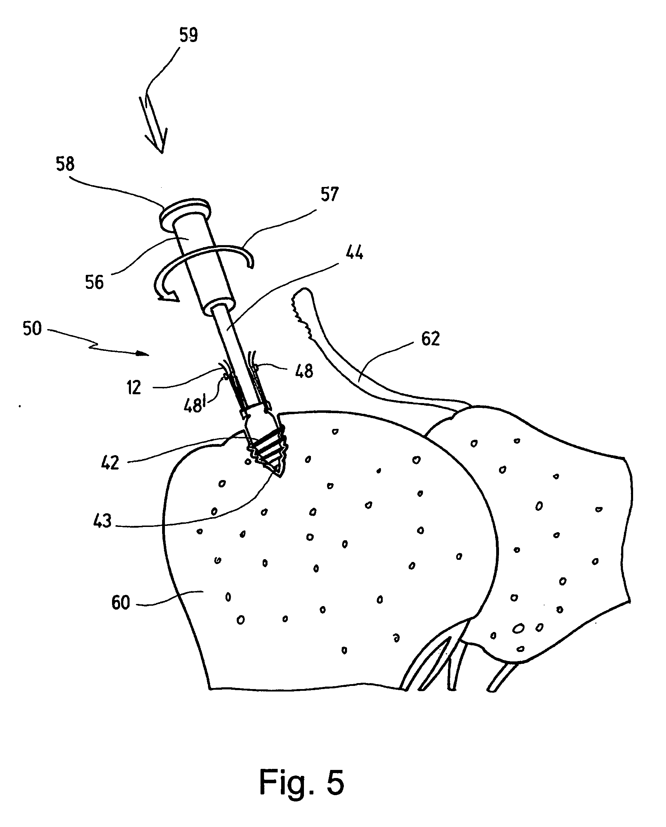

Zum Einbringen des Ankerelementes 10 wird nun das komplette Werkzeug 50, wie in Fig. 5 dargestellt, zunächst mit der Spitze 42 des Ankerelementes 10 an einer bestimmten Stelle am Knochen, beispielsweise einem Schulterknochen 60, angesetzt. Um das Ankerelement 10 in den Schulterknochen 60 hineinzudrehen, weist das Werkzeug 44 proximal einen Griff 56 auf. Die Drehung des Werkzeuges 44 ist in der Fig. 5 durch einen Pfeil 57 dargestellt. Das selbstschneidende Gewinde 43 bohrt sich dabei seinen eigenen Weg in den Knochen 60. Die Spitze 42 unterstützt das selbstschneidende Gewinde 43 dabei.

Alternativ lässt sich das Ankerelement 10 auch durch einen Schlag auf das Werkzeug 44, wie er durch einen Pfeil 59 angedeutet ist, mit einem Schlagwerkzeug zunächst linear eintreiben. Nunmehr wird durch Drehen des kompletten Werkzeuges 50 das Ankerelement 10 in den Knochen 60 eingedreht, wobei dies durch das Gewinde 43 unterstützt wird. Der Faden 12 bzw. 12' ist während dieses Vorgangs gegenüber einem Verschieben oder Verdrillen gesichert, da er an den Zapfen 48, 48' festgebunden ist.

Nach dem vollständigen Eindrehen des Ankerelements 10 in den Schulterknochen 60 wird das Werkzeug 44 nach proximal abgezogen. Dabei verbleibt das Ankerelement 10 im Knochen 60.Mit Hilfe der vorstehenden Enden des Fadens 12 bzw. 12' kann nunmehr eine Sehne 62, die vom Schulterknochen 60 abgerissen ist, wieder fixiert werden.

Ein Ankerelement 10, das auf diese Weise eng anliegend in dem Knochenmaterial eingebracht ist, kann, wenn es aus resorbierbarem Material hergestellt ist, nach und nach durch Knochenmaterial ersetzt werden, so dass dann die Sehne 62 wieder naturgetreu fixiert ist.

## Patentansprüche

1. Ankerelement zum Einbringen in einen Knochen (60) mit zumindest einem Faden (12) zum Fixieren einer Sehne (62), eines Bandes oder von Weichteilgewebe, mit zumindest einer Öffnung zum Aufnehmen des zumindest einen Fadens (12), und einer zentralen Bohrung (14) zum Aufnehmen eines Werkzeuges (44) zum Einbringen des Ankerelementes (10), wobei die zentrale Bohrung (14) in einem proximalen Abschnitt (20) des Ankerelementes (10) von einem materialstarken Mantel (22) umgeben ist, wobei das Ankerelement (10) als langgestreckter zylindrischer Körper (16), der keinen erweiterten Kopf aufweist, ausgebildet ist, der in einem distalen Abschnitt (30) in eine Verjüngung (18) übergeht, **dadurch gekennzeichnet, dass** im proximalen Abschnitt (20) des zylindrischen Körpers (16), in dem der Mantel (22) die zentrale Bohrung (14) umgibt, im Mantel (22) zwei diametral angeordnete Schlitze (24, 24') ausgebildet sind, die sich zur radialen Außenseite (36) öffnen und sich in umfänglicher Richtung im Mantel (22) erstrecken, und dass deren Flanken (32, 32'), radial nach außen gesehen, nach distal geneigt sind, wobei die zentrale Bohrung (14) und die Schlitze (24, 24') räumlich getrennt sind.

2. Ankerelement nach Anspruch 1, **dadurch gekennzeichnet, dass** die Schlitze (24, 24') Flanken (32, 32') aufweisen, die parallel verlaufen.

3. Ankerelement nach Anspruch 1, **dadurch gekennzeichnet, dass** die Schlitze (24, 24') Flanken (32, 32') aufweisen, die divergierend verlaufen.

4. Ankerelement nach Anspruch 2 oder 3, **dadurch gekennzeichnet, dass** die Flanken (32, 32') an ihren Kanten abgefast sind.

5. Ankerelement nach einem der Ansprüche 1 bis 4, **dadurch gekennzeichnet, dass** die Neigung der Schlitze (24, 24') einen Winkel von etwa 10° bis 80° aufweist.

6. Ankerelement nach einem der Ansprüche 1 bis 5, **dadurch gekennzeichnet, dass** jeder Schlitz (24, 24') einen abgerundeten Boden (34) aufweist.

7. Ankerelement nach Anspruch 6, **dadurch gekennzeichnet, dass** der abgerundete Boden (34) einen gekrümmten Verlauf aufweist.

8. Ankerelement nach Anspruch 7, **dadurch gekennzeichnet, dass** der gekrümmte Verlauf U-förmig ist.

## Claims

1. Anchor element for introduction into a bone (60), with at least one suture (12) for fixing a tendon (62), a ligament or soft tissue, with at least one opening for receiving the at least one suture (12), and with a central bore (14) for receiving a tool (44) for introducing the anchor element (10), wherein the central bore (14), in a proximal portion (20) of the anchor element (10) is surrounded by a jacket (22) of thick material, wherein the anchor element (10) is designed as an elongated cylindrical body (16) having no enlarged head, said body (16) continues in a distal section (16) as a taper (18), **characterized in that** in the proximal portion (20) of the cylindrical body (16) where the jacket (22) surrounds the central bore, two slits (24, 24') are arranged diametrically within the jacket (22), said slits open to the radial outside (36) and extend in circumferential direction within the jacket (22), and **in that** flanks (32, 32') thereof, are inclined to distal, when viewing in radial direction, wherein the central bore (14) and the slits (24, 24') are spaced apart one to another.

2. Anchor element of claim 1, **characterized in that** the slits (24, 24') have flanks (32, 32') that extend parallel one to another.

3. Anchor element of claim 1, **characterized in that** the slits (24, 24') have flanks (32, 32') that diverge.

4. Anchor element of claims 2 or 3, **characterized in that** the flanks (32, 32') are bevelled at their edges.

5. Anchor element of anyone of claims 1 through 4, **characterized in that** the inclination of the slits (24, 24') has an angle of approximately 10° to approximately 80°.

6. Anchor element of any one of claims 1 through 5, **characterized in that** each slit (24, 24') has a rounded base (34).

7. Anchor element of claim 6, **characterized in that** the rounded base (34) has a curved course.

8. Anchor element of claim 7, **characterized in that** the curved course is U-shaped.

## Revendications

1. Elément d'ancrage dévolu à l'insertion dans un os (60), comprenant au moins un fil (12) dédié au blocage à demeure d'un tendon (62), d'un ligament ou d'un tissu de partie molle ; au moins une ouverture conçue pour recevoir ledit fil (12) à présence minimale ; et un alésage central (14) conçu pour recevoir un outil (44) affecté à l'insertion de l'élément d'ancrage (10), ledit alésage central (14) étant entouré d'une enveloppe (22) à matériau épais, dans une région proximale (20) dudit élément d'ancrage (10), et ledit élément d'ancrage (10) étant réalisé sous la forme d'un corps cylindrique allongé (16) dépourvu de tête épaissie, qui fusionne dans un rétrécissement (18) dans une région distale (30), **caractérisé par le fait que** deux fentes (24, 24') diamétralement opposées, pratiquées dans l'enveloppe (22) dans la région proximale (20) du corps cylindrique (16) dans laquelle ladite enveloppe (22) entoure l'alésage central (14), s'ouvrent vers la face extérieure radiale (36) et s'étendent, dans ladite enveloppe (22), suivant la direction du pourtour ; et **par le fait que** les flancs (32, 32') desdites fentes sont inclinés vers la région distale, observés vers l'extérieur dans le sens radial, l'alésage central (14) et les fentes (24, 24') étant séparés dans l'espace.

2. Elément d'ancrage selon la revendication 1, **caractérisé par le fait que** les fentes (24, 24') sont pourvues de flancs (32, 32') s'étendant parallèlement.

3. Elément d'ancrage selon la revendication 1, **caractérisé par le fait que** les fentes (24, 24') sont pourvues de flancs (32, 32') s'étendant avec divergence.

4. Elément d'ancrage selon la revendication 2 ou 3, **caractérisé par le fait que** les flancs (32, 32') sont biseautés sur leurs arêtes.

5. Elément d'ancrage selon l'une des revendications 1 à 4, **caractérisé par le fait que** l'inclinaison des fentes (24, 24') présente un angle d'environ 10° à 80°.

6. Elément d'ancrage selon l'une des revendications 1 à 5, **caractérisé par le fait que** chaque fente (24, 24') est pourvue d'un fond arrondi (34).

7. Elément d'ancrage selon la revendication 6, **caractérisé par le fait que** le fond arrondi (34) offre un tracé courbe.

8. Elément d'ancrage selon la revendication 7, **caractérisé par le fait que** le tracé courbe revêt la forme d'un U.
